# DEMANDE DE BREVET EUROPEEN

(11) **EP 1 677 105 A1**
(43) Date de publication de la demande: **05.07.2006**
(21) Numéro de dépôt: 05358008.0
(22) Date de dépôt: 29.07.2005
(51) Int. Cl.: G01N 27/64

(54) **Procédé de marquage isotopique de produits et ses applications notamment à la lutte contre la contrefaçon**

(30) Priorité: 05.08.2004 FR 0408671
(71) Demandeur: Innovation & Chimie Fine, 04100 Manosque (FR)
(72) Inventeur: Frideling, Jean-Pierre, 04100 Manosque (FR)
(74) Mandataire: Galup, Cédric Olivier Nicolas

(57) **Abrégé**

Procédé de marquage d'un produit manufacturé, caractérisé en ce que l'on choisit au moins un composé qui est un constituant dudit produit manufacturé, et en ce que l'on ajoute à la composition dudit produit manufacturé une quantité déterminée d'un mélange d'au moins deux dérivés isotopiques, ou l'on remplace une quantité déterminée dudit produit manufacturé par une quantité déterminée équivalente d'un mélange d'au moins deux dérivés isotopiques, lesdits dérivés isotopiques étant soit des dérivés isotopiques différents d'un même composé naturel, soit des dérivés isotopiques d'au moins deux composés différents.

## Description

La présente invention concerne un procédé de marquage isotopique de produits et ses applications notamment à la lutte contre la contrefaçon.

La fraude, les copies illicites, les réclamations non vérifiables sont de plus en plus courantes et touchent aujourd'hui tous les domaines, qu'ils soient dans le domaine du commerce de luxe mais aussi de simples produits industriels comme les arômes, tabacs, parfums, colorants, encres, solutions technologiques, médicaments, produits manufacturés...). L'ensemble de ces produits est aujourd'hui la proie des contrefacteurs qui disposent de moyens financiers et techniques considérables pour les reproduire à l'identique.

Il serait donc souhaitable de disposer d'un procédé d'identification de mise en oeuvre simple, discret, rapide et modifiable à tous moments, permettant une authentification sans équivoque du produit à protéger.

Il serait également souhaitable de disposer d'un procédé de marquage universel pour tous les produits, variable à souhait sur l'initiative du fabricant, capable de suivre des lots de fabrication, le lieu de fabrication, le destinataire, etc.

Or après de longues recherches la demanderesse a découvert un procédé de marquage mettant en oeuvre des isotopes, qui répond aux besoins cités ci-dessus.

C'est pourquoi la présente invention a pour objet un procédé de marquage d'un produit manufacturé, caractérisé en ce que l'on choisit au moins un composé qui est un constituant dudit produit manufacturé, et en ce que
l'on ajoute à la composition dudit produit manufacturé une quantité déterminée d'un mélange d'au moins deux dérivés isotopiques, ou
l'on remplace une quantité déterminée dudit produit manufacturé par une quantité déterminée équivalente d'un mélange d'au moins deux dérivés isotopiques,
lesdits dérivés isotopiques étant soit des dérivés isotopiques différents d'un même composé naturel, soit des dérivés isotopiques d'au moins deux composés différents.

Pour simplifier la rédaction, on parlera simplement dans la suite de l'addition d'un dérivé isotopique.

Dans la présente demande et dans ce qui suit, le terme « produit manufacturé» désigne par exemple un objet quelconque fabriqué à partir d'un composé unique ou de préférence à partir de plusieurs composés, fusionnés en une entité unique comme un alliage métallique ou une matière plastique, ou juxtaposés comme un objet comprenant des parties métalliques et des parties en matière plastique par exemple.

Le terme «produit manufacturé» désigne aussi notamment un mélange de composés comme un colorant, un arôme et particulièrement un parfum. Le produit manufacturé peut être gazeux mais de préférence solide ou liquide, particulièrement ce dernier.

Le terme «dérivé isotopique» désigne un dérivé d'un composé naturel dont un ou plusieurs atomes sont substitués par autant d'isotope dudit atome de préférence non radioactifs.

On peut citer par exemple le carbone 13 pour substituer le carbone 12, l'oxygène 18 pour substituer l'oxygène 16, l'azote 15 pour substituer l'azote 14, et tout particulièrement le deutérium pour remplacer l'hydrogène.

Un tel dérivé isotopique produit un pic caractéristique en spectrométrie de masse.

Dans des conditions préférentielles de mise en oeuvre de l'invention, on ajoute à la composition dudit produit manufacturé une quantité déterminée d'un mélange d'au moins deux dérivés isotopiques, ou plus, avantageusement 3 dérivés isotopiques ou plus, de préférence 4 dérivés isotopiques ou plus.

Si l'on utilise deux dérivés isotopiques ou plus de composés différents, on préfère choisir des composés proches sur le graphe de la chromatographie avant spectrométrie de masse plutôt que des composés éloignés. Le terme «composés proches» désigne des composés de temps de rétention différant de moins de 50%, notamment moins de 20% l'un de l'autre.

Dans d'autres conditions préférentielles de mise en oeuvre de l'invention, la quantité du ou de chaque dérivé isotopique ci-dessus représente de 10 ppm à 10 %, de préférence de 1‰ à 5% , notamment de 5‰ à 1%, tout particulièrement de 0,1% à 1% de la quantité du même composé « naturel » présente dans le produit manufacturé. La quantité minimum du ou de chaque dérivé isotopique utilisable est déterminée par la sensibilité de l'appareil de détection utilisé.

Dans encore d'autres conditions préférentielles de mise en oeuvre de l'invention, on choisit à titre de composé qui est un constituant dudit produit manufacturé, un composé présent dans le produit manufacturé, mais en quantité mineure.

Le composé pourra représenter pondéralement par exemple avantageusement moins de 50 %, de préférence moins de 20 %, notamment moins de 10 %, tout particulièrement moins de 5 %, du produit manufacturé.

Ainsi, le marquage peut être réalisé à moindre coût, tout en restant détectable.

Le procédé de marquage d'un produit manufacturé objet de la présente invention possède de très intéressantes propriétés et qualités. Il permet notamment de fabriquer des produits manufacturés ou lots de produits manufacturés uniques, ce qui permet de les distinguer de produits manufacturés de provenance différente.

Il permet de marquer différemment différents lots ou fabrications provenant de différents sites de production.

Il permet à un producteur d'authentifier sa composition et par là de lutter efficacement contre la diffusion de contrefaçons.

Le procédé de marquage objet de la présente invention peut également servir à la reconnaissance d'un lot de fabrication. En utilisant un code différent par client, un fabricant peut éviter des réclamations abusives. Il peut être utilisé dans toute application nécessitant une reconnaissance fine et discrète de l'origine d'un produit.

On peut affecter un signe, par exemple un chiffre ou une lettre, à une proportion donnée d'un dérivé isotopique donné et obtenir ainsi un code d'identification d'un produit manufacturé, pouvant être plus ou moins élaboré selon le nombre de dérivés isotopiques ajoutés. La référence pour l'établissement d'un code peut être choisie librement, par exemple la quantité de composé ou celle du dérivé isotopique majoritaire ou minoritaire par exemple s'il y en a plusieurs.

Ainsi l'invention a aussi pour objet l'utilisation du procédé de marquage d'un produit manufacturé tel que décrit précédemment, pour l'identification dudit produit manufacturé,

Selon un mode de réalisation de l'invention, on peut identifier un produit manufacturé à l'aide d'un code établit en prédéterminant par exemple dans ledit produit manufacturé soit les quantités des différents dérivés isotopiques du mélange ajouté ou substitué, soit le rapport de la quantité d'au moins un dérivé isotopique d'un composé sur la quantité totale du mélange de dérivés isotopiques ajouté ou substitué dans le produit manufacturé ; soit le rapport de la quantité d'au moins un dérivé isotopique d'un composé sur la quantité totale dudit (ou desdits) composé(s) naturel(s) présent(s) dans le produit manufacturé ; soit le rapport de la quantité d'un dérivé isotopique d'un composé sur la quantité d'un autre dérivé isotopique du même composé ; soit le rapport de la quantité d'un dérivé isotopique d'un composé sur la quantité d'un dérivé isotopique d'un autre composé choisi arbitrairement comme référence.

Ces propriétés sont illustrées ci-après dans la partie expérimentale. Elles justifient l'utilisation du marquage par des composés isotopiques, ci-dessus décrits, dans un procédé de vérification de l'identité d'un produit manufacturé.

C'est pourquoi la présente demande a aussi pour objet un procédé de vérification de l'identité d'un produit manufacturé, éventuellement non original, contrefait ou falsifié, dont les exemplaires originaux ont préalablement été marqués par mise en oeuvre du procédé de marquage ci-dessus décrit.

Selon un mode particulier de mise en oeuvre dudit procédé de vérification celui-ci comprend les étapes suivantes :
a) on prélève un échantillon dudit produit manufacturé ;
b) on procède à une analyse par spectrométrie de masse dudit échantillon ;
c) on détermine la quantité d'un ou des dérivés isotopiques éventuellement présents dans ledit échantillon et
d) on compare ladite ou lesdites quantité(s) ou le ou les rapport(s) de la ou desdites quantité(s) déterminée(s) à l'étape c) à la ou aux quantité(s) ou au(x) rapport(s) desdites quantité(s) du même dérivé isotopique présent dans le produit manufacturé identifié original.

Il est alors possible de déduire si le produit manufacturé est original ou provient d'une source différente, par exemple une contrefaçon.

Ce procédé permet entre autre de détecter d'éventuelle contrefaçon.

Si l'on a marqué différemment des produits manufacturés originaux provenant d'une fabrication différente (millésimes ou lots ou sites de fabrication différant par exemple), on peut en déduire l'origine ou la date d'élaboration des produits.

Dans beaucoup de cas, il est possible d'utiliser directement le produit manufacturé pour l'analyse par spectrométrie de masse. C'est le cas par exemple pour des composés de masse moléculaire élevée, par exemple supérieure à 150, notamment supérieure à 200, tout particulièrement supérieure à 300, ou présentant une instabilité importante comme certains colorants ou médicaments.

Il est parfois nécessaire ou préférable d'effectuer une séparation totale ou partielle du composé et de ses dérivés isotopiques (ou des composés et de leurs dérivés isotopiques) avant d'effectuer l'analyse par spectrométrie de masse. On utilise alors une méthode de séparation non destructive préalablement à l'analyse par spectrométrie de masse. Par exemple, dans le cas de mélanges de multiples composés comme un parfum, un arôme, on pourra utiliser une chromatographie couplée ou non à la spectrométrie de masse.

La détermination des quantités relatives du ou des dérivés isotopiques pourra être effectuée directement ou à l'aide d'un étalon interne. Ledit étalon interne pourra être par exemple un dérivé isotopique d'un composé qui est un constituant dudit produit manufacturé, par exemple le dérivé isotopique présent en quantité majoritaire. Ledit étalon interne peut également être dérivé isotopique non présent dans le produit manufacturé auquel on ajoute une quantité déterminée et connue dudit composé étalon. On préférera cette dernière pratique en particulier lorsque l'on utilise deux dérivés isotopiques ou plus, de composés différents éloignés sur le graphe de spectrométrie de masse.

Les conditions préférentielles de mise en oeuvre des procédés de marquage d'un produit manufacturé ci-dessus décrites s'appliquent également aux autres objets de l'invention visés ci-dessus, notamment aux procédés de vérification de l'identité d'un produit manufacturé.

Les exemples qui suivent illustrent la présente demande.

### Exemple 1 : Marquage et codage d'une solution technologique

On souhaite identifier une suspension de 180Kg comprenant deux phases liquides non miscibles entre elles, 100Kg de phase organique, 80Kg de phase aqueuse.

La phase organique contient plusieurs composés dont 2 Kg de 1,10-dibromodécane que l'on choisit pour réaliser le marquage.

On ajoute au mélange ci-dessus
- 1,33g (soit 20% du dérivé isotopique majoritaire) d'un premier dérivé isotopique du 1,10-dibromodécane qui est le dérivé deutérié sur les positions 1 et 10 par introduction dans la molécule de 4 atomes de deutérium,
- 6,67g (dérivé isotopique majoritaire soit 100%) d'un second dérivé isotopique du 1,10-dibromodécane qui est le dérivé deutérié sur les positions 1, 5, 6 et 10 par introduction dans la molécule de 8 deutériums pour la seconde entité isotopique (entité majoritaire),
- 2,00g (soit 30% du dérivé isotopique majoritaire) d'un troisième dérivé isotopique du 1,10-dibromodécane qui est le dérivé deutérié sur les positions 1, 3, 4, 7, 8 et 10 par introduction dans la molécule de 12 deutériums.

La quantité du mélange de ces 3 entités isotopiques représente 5‰ (10g) du 1,10-dibromodécane non isotopique initialement contenu dans la phase organique.

On attribue pour chaque dérivé la lettre A à la présence de 0% du dérivé isotopique par rapport à la quantité du dérivé isotopique majoritaire servant de référence,(100%), la lettre B à la présence de 5%, la lettre C à la présence de 10%, et ainsi de suite. On en fait de même pour le second et le troisième dérivé isotopique du 1,10-dibromodécane.

Les rapports des quantités de ces dérivés isotopiques à la quantité de dérivé isotopique majoritaire, sont de 20%/100%/30%. Par référence à ce qui précède, le code du lot est caractérisé par les trois lettres EUG.

Ce marquage identifie et constitue le code de ce lot.

### Exemple 2 : Identification d'une solution technologique

On analyse par GC-MS un échantillon du lot préparé à l'exemple 1. L'analyse montre 3 fragments caractéristiques du 1,10-dibromodécane marqué par le deutérium et plus particulièrement les fragments M/S 225, 229, 233. Le rapport des intensités des pics caractéristiques de ces fragments observé et mesuré sur le diagramme d'analyse est de 20%/100%/30%.

Par référence au codage ci-dessus, le code du lot est caractérisé par les trois lettres EUG ; il authentifie le lot.

### Exemple 3 : Marquage et codage d'une composition odorante

On souhaite identifier une suspension de 1000Kg de composition odorante de composition suivante :

| Composant | Poids ‰ |
|---|---|
| Acétate de benzyle | 20 |
| Acétate de géranyle | 20 |
| Acétate de linalyle | 160 |
| 2-Méthyldecanal 10% | 20 |
| Dihydromyrcénol | 130 |
| Habanolides® | 20 |
| Huile essentielle de verveine des Indes (Lemon grass) | 15 |
| Limette | 30 |
| Liminal | 5 |
| Linalool | 90 |
| Hedione® | 60 |
| Muscénone | 20 |
| Z-3-Décénal 10% | 20 |
| Oxane 10% | 20 |
| Rose oxyde 10% | 20 |
| Huile essentielle d'orange | 290 |
| Trans-2,6,6-triméthyl-3-cyclohexène-1-carboxylate de méthyle | 60 |

On choisit l'acétate de benzyle pour réaliser le marquage.

On ajoute au mélange ci-dessus
- 0,4g (soit 10% du dérivé isotopique majoritaire) d'un premier dérivé isotopique de l'acétate de benzyle qui est le dérivé deutérié sur le carbone benzylique par introduction dans la molécule de 2 deutériums,
- 3,2g (soit 80% du dérivé isotopique majoritaire) d'un second dérivé isotopique de l'acétate de benzyle qui est le dérivé deutérié sur l'acétate par introduction dans la molécule de 3 deutériums pour la seconde entité isotopique (entité majoritaire),
- 1,6g (soit 40% du dérivé isotopique majoritaire) d'un troisième dérivé isotopique de l'acétate de benzyle qui est le dérivé deutérié sur le cycle benzénique par introduction dans la molécule de 5 deutériums.
- 4,0g (dérivé isotopique majoritaire soit 100%) d'un quatrième dérivé isotopique de l'acétate de benzyle qui est le dérivé deutérié marqué uniformément sur le benzyle par introduction dans la molécule de 7 deutériums,
- 0,8g (soit 20% du dérivé isotopique majoritaire) d'un cinquième dérivé isotopique de l'acétate de benzyle qui est le dérivé deutérié marqué uniformément sur l'acétate de benzyle par introduction dans la molécule de 10 deutériums.

La quantité du mélange de ces 5 entités isotopiques représente 1 % (10g) de l'acétate de benzyle non isotopique initialement contenu dans la phase organique.

Les rapports des quantités de ces dérivés isotopiques à la quantité de dérivé isotopique majoritaire sont de 10%/80%/40%/100%/20%. Par référence au codage de l'exemple 1, le code du lot est caractérisé par les 5 lettres CQIUE.

Ce marquage identifie et constitue le code de ce lot.

### Exemple 4 : Identification d'une composition odorante

On analyse par GC-MS un échantillon du lot préparé à l'exemple 3. L'analyse montre 5 fragments caractéristiques du benzyle acétate marqué par le deutérium et plus particulièrement les fragments M/S 152, 153, 155, 157, 161. Le rapport des intensités de ces fragments est de 10%/80%/40%/100%/20%. Par référence au codage de l'exemple 1, le code est caractérisé par les cinq lettres CQIUE ; il authentifie le lot.

### Exemple 5 : Marquage et codage d'un tabac

On souhaite identifier 1000Kg de tabac du type tabac de Virginie, parfumé avec 30kg de composition odorante de composition suivante

| Composant | Poids en Kg |
|---|---|
| Ethanol 96% V/V | 0,27 |
| Vanillin | 0,75 |
| Huile essentielle d'orange | 0,030 |
| gamma-Octalactone | 0,075 |
| Sucre caramélisé | 3,45 |
| Extrait de Fenugrec | 1,455 |
| Méthyl cyclopentènolone | 0,24 |
| Acide phénylacétique | 1,2 |
| 1,2-propylène glycol USP/EP | 10,5 |
| Eau | 2,4 |
| Vinaigre de vin blanc 10% | 0,15 |
| Huile de Livèche | 0,21 |
| Sirop de sucre inverti 81% | 2,88 |
| Extrait de prune | 3,9 |
| Extrait de cacao | 2,205 |
| Acétate d'isoamyle | 0,18 |
| Cinnamate de méthyle | 0,105 |

On choisit le cinnamate de méthyle pour réaliser le marquage.

On ajoute au mélange ci-dessus
- 5,25g d'un dérivé isotopique de cinnamate de méthyle qui est le dérivé deutérié sur le carbone méthylique par introduction dans la molécule de 3 deutériums.

### Exemple 6 : Identification d'un tabac

On analyse par GC-MS un échantillon du lot préparé à l'exemple 5.

On prépare tout d'abord un étalon interne qui est le dérivé de cinnamate de méthyle deutérié uniformément sur le cinnamyle par introduction dans la molécule de 7 deutériums.

On ajoute 5,25ml d'une solution alcoolique contenant 1mg/ml de l'étalon interne (5,25mg étant la quantité de cinnamate de méthyle deutérié sur le carbone méthylique attendue dans 100g de tabac original), à 100 grammes de tabac.

Après mélange, le mélange est finement broyé. Il est extrait par 3 fois 100ml de dichlorométhane. Le résidu obtenu après évaporation du solvant est analysé par GC-MS. On mesure l'intensité des deux fragments caractéristiques du cinnamate de méthyle marqué par le deutérium et plus particulièrement les fragments M/S 165, 169.

Le rapport des intensités de ces deux fragments est égal à 1.

L'absence du pic 165 et ou une variation sensible de l'intensité de ce pic prouverait la fraude ou des manipulations sur le produit.

### Exemple 7 : Marquage et codage d'un arôme alimentaire

On souhaite identifier 1000Kg d'arôme banane sans alcool, de composition suivante :

| Composant | Poids en kg |
|---|---|
| Acétate d'isoamyle | 10,0 |
| Butyrate d'isoamyle | 2,0 |
| Butyrate d'éthyle | 0,5 |
| Acétate d'éthyle | 1,0 |
| Valérianate d'isoamyl | 3,0 |
| Vanilline | 0,5 |
| Eugénol | 0,1 |
| Cis 3-Hexenol | 0,5 |
| Monopropylène Glycol | 82,4 |

On choisit l'acétate d'éthyle pour réaliser le marquage.

On ajoute au mélange ci-dessus
- 1,5g (soit 30% du dérivé isotopique majoritaire) d'un premier dérivé isotopique de l'acétate d'éthyle qui est le dérivé deutérié sur le carbone 1 de l'éthyle par introduction dans la molécule de 2 deutériums,
- 5g (dérivé isotopique majoritaire soit 100%) d'un second dérivé isotopique de l'acétate d'éthyle qui est le dérivé deutérié sur l'acide acétique par introduction dans la molécule de 3 deutériums.
- 1 g (soit 20% du dérivé isotopique majoritaire) d'un troisième dérivé isotopique de l'acétate d'éthyle qui est le dérivé uniformément deutérié sur l'éthyle par introduction dans la molécule de 5 deutériums.
- 2,5g (soit 50% du dérivé isotopique majoritaire) d'un quatrième dérivé isotopique de l'acétate d'éthyle qui est le dérivé uniformément deutérié marqué uniformément sur l'acétate d'éthyle par introduction dans la molécule de 8 deutériums,

La quantité du mélange de ces 4 entités isotopiques représente 1‰ (10g) de l'acétate d'éthyle non isotopique initialement contenu dans l'arôme.

Les rapports des quantités de ces dérivés isotopiques à la quantité de dérivé isotopique majoritaire sont de 30%/100%/20%/50%. Par référence au codage de l'exemple 1, le code du lot est caractérisé par les 4 lettres GUEK. Ce marquage identifie et constitue le code de ce lot.

### Exemple 8 : Identification d'un arôme alimentaire

On analyse par GC-MS un échantillon du lot préparé à l'exemple 7. La solution est directement injectée dans le chromatographe et l'on analyse par masse le pic d'acétate d'éthyle. On mesure l'intensité des quatre fragments caractéristiques de l'acétate d'éthyle marqué par le deutérium et plus particulièrement les fragments M/S 90, 91, 93, 96.

Le rapport des intensités de ces quatre fragments est égal à 30%/100%/20%/50%.

Par référence au codage de l'exemple 1, le code est caractérisé par les quatre lettres GUEK ; il authentifie le lot testé.

### Exemple 9 : Marquage et codage d'une solution de colorant pour encres

On souhaite identifier 200kg de dispersion de colorant pour encre de composition suivante :

| Composant | Poids en kg |
|---|---|
| Regal® 660 carbon black | 9,0 |
| Solsperse® 5000 | 0,22 |
| Solsperse® 24000 | 2,26 |
| DPGDA (Diacrylate de dipropylène glycol) | 91,88 |
| Triacrylate de triméthylol propane éthoxylé | 28,0 |
| Irgacure® 369:1700:819 (1.0 :1.0 :1.0) | 18,0 |
| Diacrylate de néopentyl glycol propyloxylé | 44,04 |
| DPHA (hexaacrylate de dipentaérythritol) | 6,0 |
| Addid® 300 | 0,6 |

On choisit l'hexaacrylate de dipentaérythritol pour réaliser le marquage. Ce produit peut être proposé avec 4 masses différentes provenant du marquage de l'acide acrylique par 3 deutériums. Pour ce faire, on a réalisé la synthèse des mono, di, tri, tétra acrylate-D3 de l'hexaacrylate de dipentaérythritol.

On ajoute au mélange ci-dessus
- 0,4g (soit 10% du dérivé isotopique majoritaire) d'un premier dérivé isotopique de l'hexaacrylate de dipentaérythritol marqué par un acide acrylique trideutérié, soit trois deutériums.
- 0,4g (soit 10% du dérivé isotopique majoritaire) d'un second dérivé isotopique de l'hexaacrylate de dipentaérythritol marqué par deux acides acryliques trideutériés, soit six deutériums.
- 4g (dérivé isotopique majoritaire soit 100%)) d'un troisième dérivé isotopique de l'hexaacrylate de dipentaérythritol marqué par trois acides acryliques trideutériés, soit neuf deutériums.
- 1,2g (soit 30% du dérivé isotopique majoritaire) d'un quatrième dérivé isotopique de l'hexaacrylate de dipentaérythritol marqué par quatre acides acryliques trideutériés, soit douze deutériums.

La quantité du mélange de ces 4 entités isotopiques représente 1‰ (6g) de l'hexaacrylate de dipentaérythritol non isotopique initialement contenu dans la composition.

Le rapport des quantités de ces dérivés isotopiques est de 10%/10%/100%/30%. Par référence au codage de l'exemple 1, le code du lot est caractérisé par les 4 lettres CCUG.

Ce marquage identifie et constitue le code de ce lot.

### Exemple 10 : Identification d'une solution de colorant pour encres

Un échantillon du lot préparé à l'exemple 9 est chromatographié sur une petite colonne de silice (SILICA BP SUP). La fraction contenant l'hexaacrylate de dipentaérythritol est ensuite analysée par GC-MS. L'analyse montre 4 fragments caractéristiques de l'hexaacrylate de dipentaérythritol marqué par le deutérium et plus particulièrement les fragments M/S 581, 584, 587, 590. Le rapport des intensités de ces fragments est de 10%/10%/100%/30%. Par référence au codage de l'exemple 1, le code est caractérisé par les cinq lettres CCUG ; il authentifie le lot.

## Revendications

1. Procédé de marquage d'un produit manufacturé, **caractérisé en ce que** l'on choisit au moins un composé qui est un constituant dudit produit manufacturé, et **en ce que**
l'on ajoute à la composition dudit produit manufacturé une quantité déterminée d'un mélange d'au moins deux dérivés isotopiques, ou
l'on remplace une quantité déterminée dudit produit manufacturé par une quantité déterminée équivalente d'un mélange d'au moins deux dérivés isotopiques,
lesdits dérivés isotopiques étant soit des dérivés isotopiques différents d'un même composé naturel, soit des dérivés isotopiques d'au moins deux composés différents.

2. Procédé de marquage selon la revendication 1, **caractérisé en ce que** la quantité du ou de chaque dérivé isotopique représente de 10 ppm à 10 % du composé naturel qui est un constituant dudit produit manufacturé.

3. Procédé de marquage selon l'une quelconque des revendications 1 ou 2, **caractérisé en ce que** le composé choisi est un composé présent dans le produit manufacturé mais représentant pondéralement moins de 20% dudit produit manufacturé.

4. Procédé de marquage selon l'une quelconque des revendications 1 à 3, **caractérisé en ce que** le dérivé isotopique est un dérivé d'un composé naturel dont un ou plusieurs atomes sont substitués par autant d'isotope dudit atome.

5. Procédé de marquage selon l'une quelconque des revendications 1 à 4, **caractérisé en ce que** l'isotope est choisi parmi le deutérium, le carbone 13, l'oxygène 18, l'azote 15, préférentiellement le deutérium.

6. Utilisation du procédé de marquage d'un produit manufacturé tel que décrit dans l'une quelconque des revendications 1 à 5, pour l'identification dudit produit manufacturé.

7. Utilisation selon la revendication 6, **caractérisé en ce que** l'on prédétermine dans ledit produit manufacturé
soit les quantités des différents dérivés isotopiques du mélange ajouté ou substitué
soit le rapport de la quantité d'au moins un dérivé isotopique d'un composé sur la quantité totale du mélange de dérivés isotopiques ajouté ou substitué dans le produit manufacturé ;
soit le rapport de la quantité d'au moins un dérivé isotopique d'un composé sur la quantité totale dudit (ou desdits) composé(s) naturel(s) présent(s) dans le produit manufacturé ;
soit le rapport de la quantité d'un dérivé isotopique d'un composé sur la quantité d'un autre dérivé isotopique du même composé ;
soit le rapport de la quantité d'un dérivé isotopique d'un composé sur la quantité d'un dérivé isotopique d'un autre composé choisi arbitrairement comme référence.

8. Procédé de vérification de l'identité d'un produit manufacturé dans lequel l'exemplaire original a préalablement été marqué par mise en oeuvre du procédé de marquage tel que défini à l'une des revendications 1 à 5 et dans lequel on procède à une analyse par spectrométrie de masse d'un échantillon dudit produit manufacturé.

9. Procédé de vérification selon la revendication 8, comprenant les étapes suivantes :
a) on prélève un échantillon dudit produit manufacturé ;
b) on procède à une analyse par spectrométrie de masse dudit échantillon
c) on détermine la quantité d'un ou des dérivés isotopiques éventuellement présents dans ledit échantillon et
d) on compare ladite ou lesdites quantité(s) ou le ou les rapport(s) de la ou desdites quantité(s) déterminée(s) à l'étape c) à la ou aux quantité(s) ou au(x) rapport(s) desdites quantité(s) du même dérivé isotopique d'un produit manufacturé identifié original.
